# FASCICULE DE BREVET EUROPEEN

(11) **EP 0 784 467 B1**
(45) Date de publication et mention de la délivrance du brevet: **11.11.1998**
(21) Numéro de dépôt: 95943257.6
(22) Date de dépôt: 21.12.1995
(51) Int. Cl.: A61K 9/00

(54) **FORME GALENIQUE UNITAIRE DESTINEE A LA VOIE VAGINALE UTILISABLE COMME CONTRACEPTIF LOCAL ET/OU POUR COMBATTRE LES MALADIES SEXUELLEMENT TRANSMISSIBLES ET/OU LES VIH**
EINZELDOSIERUNGSFORM ZUR VAGINALEN VERABREICHUNG, VERWENDBAR ALS LOKALKONTRAZEPTIUUM UND/ODER ZUR BEKAMPFUNG VON SEXUELL UEBERTRAGENEN KRANKHEITEN UND/ODER UIH
UNITARY VAGINAL DOSAGE FORM USEFUL AS A LOCAL CONTRACEPTIVE AND/OR FOR COMBATTING SEXUALLY TRANSMITTED DISEASES AND/OR HIV

(30) Priorité: 22.12.1994 FR 9415501
(43) Date de publication de la demande: 23.07.1997
(73) Titulaire: LABORATOIRE INNOTHERA Société Anonyme, 94111 Arcueil (FR)
(72) Inventeur: MEIGNANT, Catherine, 75010 Paris (FR)
(74) Mandataire: Dupuis-Latour, Dominique
(86) Numéro de dépôt international: FR9501712
(87) Numéro de publication internationale: WO9619195

(56) Documents cités:
- EP-A- 0 359 402
- FR-A- 2 372 635

## Description

La présente invention concerne une forme galénique unitaire de type capsule molle, c'est-à-dire comprenant, d'une part, une enveloppe externe comprenant de la gélatine et, d'autre part, une phase interne liquide ou semi-liquide non aqueuse contenant un principe actif spermicide en solution.

Plus particulièrement, elle concerne une forme galénique de ce type destinée à la voie vaginale, utilisable comme contraceptif local et/ou pour combattre les maladies sexuellement transmissibles et/ou les VIH.

Il existe à l'heure actuelle de nombreux produits spermicides sous forme d'ovules, de crèmes, d'éponges imprégnées de crème ou de solutions et de comprimés pour la contraception locale. Les agents spermicides sont tous, par nature des tensio-actifs (agents de surface).

Par exemple, les US-A-4 983 393 et US-A- 5 069 906 décrivent une composition de gel solidifié qui se dissout en présence des fluides vaginaux, le EP-A-0 587 431 décrit un ovule se dissolvant dans le vagin, comprenant une mousse lyophilisée hydrosoluble et un spermicide et le US-A-4 187 286 décrit un ovule comprenant un mélange de polyéthylène glycol, un spermicide, un agent épaississant, un agent effervescent et de l'acide alginique. Le EP-A-0 359 402 décrit un ovule contraceptif à effet prolongé comprenant un spermicide, une gomme polymère, un agent dispersant et, comme excipient majeur, un polyoxyéthylène glycol (PEG), mais il s'agit d'une forme galénique homogène et solide, dont la composition ne pourrait être adaptée à une phase interne de capsule molle compte tenu du caractère fortement hydrophile du PEG qui déstructurerait spontanément la phase externe de gélatine.

La forme galénique dite "capsule molle" est, quant à elle, bien connue, avec une phase externe initialement solide - donc aisée à manipuler et à mettre en place - comportant de la gélatine, notamment constituée de gélatine et de glycérine, enfermant une phase interne liquide ou semi-liquide renfermant un agent thérapeutique.

Par exemple, le FR-A-2 372 635 décrit une capsule molle dont la phase interne est majoritairement constituée d'un tensio-actif, donc incompatible avec une utilisation par voie vaginale pour des raisons d'intolérance par la muqueuse ; le EP-A-0 121 321 décrit une capsule molle comprenant un principe actif dissous ou en suspension et une composition empêchant la fragilisation de la capsule molle.

Par ailleurs, le développement ces dernières années des maladies sexuellement transmissibles (MST), dont notamment les infections par les VIH, a conduit à un usage de plus en plus large des préservatifs.

Il est cependant recommandé d'associer l'usage du préservatif à celui d'un agent lubrifiant approprié, c'est-à-dire qui ne dégrade pas les propriétés de résistance mécanique du préservatif et n'augmente pas sa porosité du fait d'une attaque du latex.

Par exemple, le EP-A-0 457 127 décrit un lubrifiant à base d'huile de silicone pour traiter le latex des préservatifs, le EP-A-0 475 664 décrit une composition lubrifiante et son utilisation avec des préservatifs, et le FR-A-2 666 587 décrit un lubrifiant comprenant un polydiméthylsiloxane.

D'autre part, il est également recommandé d'associer pour une plus grande sécurité l'usage du préservatif à celui d'un produit spermicide (lorsque le préservatif est utilisé à des fins de contraception) et/ou à celui d'un agent protecteur à l'égard des infections sexuellement transmissibles (lorsque le préservatif est utilisé à des fins de prévention des MST).

L'un des buts de l'invention est de proposer une forme galénique qui réponde à ces différents besoins grâce à une forme unitaire destinée à la voie vaginale, offrant les avantages connus de la capsule molle, qui présente des propriétés à la fois spermicides, antiseptiques et lubrifiantes et qui soit de plus compatible de façon sûre avec les préservatifs, sans risque de détérioration du latex de caoutchouc de ces derniers.

En effet, selon des études bibliographiques, un grand nombre de formulations administrées par voie vaginale (gels, ovules, capsules molles et analogues) ont la propriété d'altérer les caractéristiques physiques du latex de caoutchouc des préservatifs.

Un problème majeur réside en effet dans le fait que le latex de caoutchouc des préservatifs perd ses propriétés, notamment de résistance à l'éclatement, lorsqu'il est mis au contact d'un grand nombre de formulations administrées par voie vaginale.

D'un autre côté, la forme galénique doit être bien tolérée, stable, et galéniquement acceptable.

Elle doit répondre à de nombreuses exigences, à savoir :
- procurer une bioadhésion pour éviter au maximum le phénomène d'écoulement,
- assurer une compatibilité des véhicules avec le principe actif,
- favoriser l'homogénéisation de la phase interne avec les sécrétions vaginales en apportant un certain caractère hydrophile, tout en assurant que ce caractère hydrophile soit compatible avec la phase externe comportant de la gélatine afin de ne pas la dénaturer (autrement la capsule fondrait d'elle-même avant même d'être utilisée),
- choisir des ingrédients compatibles à la fois avec la phase externe et le latex de caoutchouc des préservatifs.

La présente invention résout les problèmes mentionnés ci-dessus en proposant une forme galénique unitaire du type précité, caractérisée en ce que le principe actif est constitué d'un spermicide et en ce que la phase interne comporte, outre le principe actif : en proportion majoritaire, un agent lipophile compatible avec le latex de caoutchouc d'un préservatif; en proportion minoritaire, au moins un agent hydrodispersible ; au moins un agent de bioadhésion; et au moins un agent gélifiant de l'agent lipophile.

Un certain nombre de caractéristiques subsidiaires avantageuses sont visées dans les sous-revendications 2 à 14.

L'invention a également pour objet un procédé d'obtention de la forme galénique unitaire, qui est caractérisé en ce qu'il consiste : a) à ajouter sous agitation une solution de chlorure de benzalkonium à de l'huile de silicone ; b) à incorporer au mélange a) du polyoxyéthylène glycol 400 et du 7-glycéryl-cocoate de polyoxyéthylène glycol sous forme liquide ; c) à mélanger le produit b) selon une durée suffisante pour obtenir une bonne homogénéisation ; d) à ajouter au produit c) de l'hydroxypropylcellulose ; e) à mélanger le produit d) ; f) à incorporer de la silice ; et g) à agiter jusqu'à obtention du produit final, qui est introduit dans l'enveloppe comprenant de la gélatine.

Divers avantages et caractéristiques de la présente invention ressortiront des exemples de réalisation ci-après.

Dans tous les exemples de la présente invention on mentionne le contenu de la phase interne, étant bien entendu que celle-ci est introduite dans une enveloppe externe comportant de la gélatine, notamment une enveloppe externe de gélatine/glycérine connue sous la dénomination de "capsule molle".

Les exemples sont donnés, sauf indication contraire, pour une quantité de 100 grammes.

### Exemple 1

| | |
|---|---|
| Chlorure de benzalkonium, solution à 50 % | 2,65 g |
| Tristéarate d'aluminium | 2 g |
| Méthylcellulose | 10 g |
| Glycérides polyoxyéthylénés (Labrafil CS 1944®) | 10 g |
| Huile de silicone | q.s. 100 g |
| (soit huile 75,35 % et agent hydrodispersible 10 %) | |

### Exemple 2

| | |
|---|---|
| Nonoxynol 9 | 8 g |
| Alcool cétylique | 5 g |
| Acide carboxyvinylique (Carbopol 944 P®) | 2 g |
| Hydroxypropylméthylcellulose | 5 g |
| Polyoxyéthylène glycol 400 | 7 g |
| Huile de silicone | q.s. 100 g |
| (soit huile 73 % et agent hydrodispersible 7 %) | |

### Exemple 3

| | |
|---|---|
| Ether laurylique (Laureth 9) | 7 g |
| Silice | 5 g |
| Acide carboxyvinylique (Carbopol 944 P®) | 2 g |
| Monolaurate de polyoxyéthylène glycol 400 | 4 g |
| Glycérides polyoxyéthylénés (Labrafil CS 1944®) | 8 g |
| Huile de silicone | q.s. 100 g |
| (soit huile 74 % et agents hydrodispersibles 12 %) | |

### Exemple 4

| | |
|---|---|
| Chlorure de benzalkonium, solution à 50 % | 2 g |
| Silice | 5 g |
| Méthylcellulose | 8 g |
| Huile de ricin polyoxyéthylénée | 5 g |
| 7-glycéryl-cocoate de polyoxyéthylène glycol (Cétiol HE®) | 8 g |
| Huile de silicone | q.s. 100 g |
| (soit huile 72 % et agents hydrodispersibles 13 %) | |

### Exemple 5

| | |
|---|---|
| Chlorure de benzéthonium | 1 g |
| Monostéarate d'aluminium | 2 g |
| Hydroxypropylméthylcellulose | 7 g |
| 7-glycéryl-cocoate de polyoxyéthylène glycol (Cétiol HE®) | 9 g |
| Polyoxyéthylène glycol 400 | 6 g |
| Huile de silicone | q.s. 100 g |
| (soit huile 75 % et agents hydrodispersibles 15 %) | |

### Exemple 6

| | |
|---|---|
| Octoxynol 9 | 6 g |
| Alcool cétylique | 5 g |
| Acide carboxyvinylique (Carbopol EX 55®) | 3 g |
| Glycérides polyoxyéthylénés (Labrafil CS 1944®) | 7 g |
| Huile de silicone | q.s. 100 g |
| (soit huile 79 % et agent hydrodispersible 7 %) | |

### Exemple 7

| | |
|---|---|
| Chlorure de méthylbenzéthonium | 1,5 g |
| Silice | 6 g |
| Carboxyméthylcellulose | 7 g |
| Monolaurate de polyoxyéthylène glycol 400 | 4 g |
| Éthyldiéthylèneglycol rectifié (Transcutol®) | 2 g |
| Huile de silicone | q.s. 100 g |
| (soit huile 79,5 % et agents hydrodispersibles 6 %) | |

### Exemple 8

| | |
|---|---|
| Chlorure de benzalkonium, solution à 50 % | 2,52 g |
| Tristéarate d'aluminium | 1,5 g |
| Méthylcellulose | 5 g |
| Glycérides polyoxyéthylénés (Labrafil CS 1944®) | 8 g |
| Polyoxyéthylène glycol 200 | 4 g |
| Huile de silicone | q.s. 100 g |
| (soit huile 78,98 % et agents hydrodispersibles 12 %) | |

### Exemple 9

| | |
|---|---|
| Chlorure de benzalkonium, solution à 50 % | 2,52 g |
| Silice | 6 g |
| Hydroxypropylcellulose | 7 g |
| 7-glycéryl-cocoate de polyoxyéthylène glycol (Cétiol HE®) | 10 g |
| Polyoxyéthylène glycol 400 | 5 g |
| Huile de silicone | q.s. 100 g |
| (soit huile 69,48 % et agents hydrodispersibles 15 %) | |

La forme galénique unitaire selon l'invention telle qu'illustrée par les exemples ci-dessus présente l'avantage d'être à la fois lubrifiante et spermicide, tout en ayant également des propriétés efficaces contre les maladies sexuellement transmissibles et les VIH, ceci en restant parfaitement compatible avec le latex de caoutchouc des préservatifs.

Les essais de compatibilité entre la forme galénique unitaire de l'invention et le latex de caoutchouc des préservatifs selon la norme AFNOR NF S 97-031 de mai 1993 ont montré qu'il ne se produisait en aucune façon une dégradation significative des propriétés des préservatifs, notamment de leur résistance à l'éclatement.

Des essais ont été mis en oeuvre au Laboratoire national d'essais sur des échantillons présentés, à savoir 50 capsules molles correspondant à la formulation de l'exemple 9 ci-dessus.

La procédure d'essais était la suivante : un essai d'éclatement (mesure du volume et de la pression d'éclatement) tel que défini dans la norme NF S 97-031 de mai 1993, § 5.2 a été réalisé sur 20 préservatifs masculins de chaque référence, dans les conditions ci-dessous :
- préservatifs masculins prêts à l'emploi (lubrifiés et conditionnés sous emballage individuel);
- préservatifs masculins mêmes conditions que ci-dessus, ayant en plus une quantité de 500 ± 30 mg de produit référencé ci-dessus appliqué au pinceau pendant 30 minutes.

Les trois références de préservatifs masculins utilisés pour les essais étaient :
- Prophyltex "SN Spécial", surface lisse, n° de lot : 231 005 - épaisseur : 60 µm ;
- Prophyltex "Stymulève", surface texturée, n° de lot : 341 201 - épaisseur : 60 µm ;
- Manix "Contact", surface lisse, n° de lot : LD 103 F12 - épaisseur : 50 µm.

Les résultats obtenus ont été les suivants :

| Référence préservatif | Volume d'éclatement (dm³) | | Pression d'éclatement (kPa) | |
|---|---|---|---|---|
| | non enduit | enduit | non enduit | enduit |
| Prophyltex "SN Spécial" : | | | | |
| valeur moyenne | 30,2 | 40,2 | 1,87 | 1,92 |
| écart-type | 4,6 | 1,9 | 0,18 | 0,08 |

| Prophyltex "Stymulève" : | | | | |
|---|---|---|---|---|
| valeur moyenne | 37,9 | 32,2 | 1,65 | 1,60 |
| écart-type | 5,2 | 8,2 | 0,09 | 0,10 |

| Manix "Contact" : | | | | |
|---|---|---|---|---|
| valeur moyenne | 37,9 | 40,4 | 1,56 | 1,46 |
| écart-type | 5,2 | 5,1 | 0,08 | 0,12 |

Ces essais permettent de conclure à une absence de modification significative du volume et de la pression d'éclatement avant et après application d'une quantité de produit selon l'invention.

Pour satisfaire aux concepts de bioadhésion de la forme galénique de l'invention et éviter au maximum le phénomène d'écoulement, la phase interne renferme dans les exemples de la présente invention des polymères bioadhésifs biocompatibles ayant la propriété d'incorporer au maximum l'humidité pour augmenter la viscosité de la forme galénique unitaire de l'invention et pour prolonger ainsi le maintien de cette forme *in situ*.

La compatibilité des véhicules avec l'agent de surface cationique constituant le principe actif est obtenue par l'utilisation d'excipients non ioniques.

Pour satisfaire aux concepts de bioadhésion, on introduit des polymères non ioniques compatibles avec la phase interne, et ces polymères non ioniques sont de préférence des dérivés cellulosiques.

Le phénomène d'écoulement par gélification de la phase interne non aqueuse est évité au maximum par l'utilisation d'un agent gélifiant de l'agent lipophile.

Dans l'invention, au moins un des ingrédients de la phase interne favorise l'homogénéisation du dérivé lipophile qui est le constituant essentiel de la phase grasse avec les sécrétions vaginales en apportant un certain caractère hydrophile à la forme galénique unitaire. Ce caractère doit néanmoins rester compatible avec la phase externe car, en effet, sous l'influence d'une hydrophilie interne trop importante, la trame externe de gélatine serait détruite.

Le constituant lipophile de l'invention et compatible avec l'agent de surface, avec l'enveloppe comportant de la gélatine et surtout avec le latex de caoutchouc des préservatifs. Dans la présente invention, l'agent lipophile utilisé est une huile de silicone, notamment du polydiméthylsiloxane répondant à la formule : 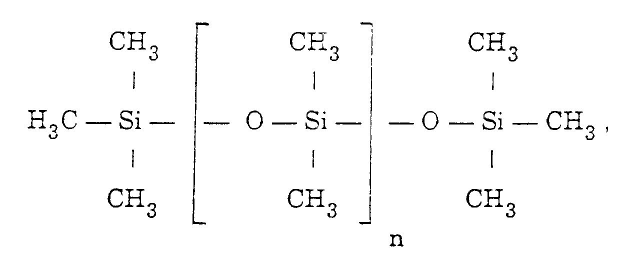 dans laquelle n est compris entre 400 et 1200.

Il s'agit d'un liquide limpide, incolore, inodore, de diverses viscosités, pratiquement insoluble dans l'eau et dans le méthanol, miscible à l'acétate d'éthyle, au chloroforme, à l'éther, à la méthyléthylcétone, au tétrachlorure de carbone et au toluène.

L'huile de silicone utilisée, qui est du diméthylpolysiloxane de type Diméticone 1000®, est conforme à la pharmacopée française dixième édition et à la pharmacopée européenne deuxième édition. La viscosité est comprise entre 950.10⁻⁶ et 1050.10⁻⁶ m²/s (950 et 1050 centistokes).

La présente invention concerne donc une forme galénique unitaire destinée à la voie vaginale sous forme d'une capsule molle, particulièrement appropriée pour être utilisée avec le latex de caoutchouc des préservatifs, qui procure une action contraceptive avec des propriétés efficaces contre les maladies sexuellement transmissibles et les VIH.

## Revendications

1. Forme galénique unitaire destinée à la voie vaginale utilisable comme contraceptif local et/ou pour combattre les maladies sexuellement transmissibles et/ou les VIH, comprenant, d'une part, une enveloppe externe comportant de la gélatine et, d'autre part, une phase interne liquide ou semi-liquide non aqueuse contenant un principe actif en solution, caractérisée en ce que le principe actif est constitué d'un spermicide et en ce que la phase interne comporte, outre le principe actif : en proportion majoritaire, un agent lipophile compatible avec le latex de caoutchouc d'un préservatif; en proportion minoritaire, au moins un agent hydrodispersible ; au moins un agent de bioadhésion ; et au moins un agent gélifiant de l'agent lipophile.

2. Forme galénique unitaire selon la revendication 1, caractérisée en ce que le spermicide est choisi parmi le chlorure de benzalkonium, le chlorure de benzéthonium, le chlorure de cétyl pyridinium, le chlorure de méthylbenzéthonium, le bromure de tétradécyltriméthyl ammonium, le bromure de benzalkonium, les éthers nonylphényliques, les éthers lauryliques, les octoxynols.

3. Forme galénique unitaire selon la revendication 2, caractérisée en ce que le spermicide est plus particulièrement de nature cationique.

4. Forme galénique unitaire selon la revendication 3, caractérisée en ce que le spermicide est le chlorure de benzalkonium.

5. Forme galénique unitaire selon la revendication 1, caractérisée en ce que l'agent lipophile compatible avec le latex de caoutchouc d'un préservatif est une huile de silicone.

6. Forme galénique unitaire selon la revendication 1, caractérisée en ce que l'agent hydrodispersible est un composé non ionique non tensio-actif.

7. Forme galénique unitaire selon la revendication 6, caractérisée en ce que l'agent hydrodispersible non ionique non tensio-actif est choisi parmi le groupe comprenant les esters d'acides gras et de polyols, l'éther d'alcool laurique et de polyéthylène glycol, l'huile de ricin polyoxyéthylénée, les glycérides polyoxyéthylénés, les polyoxyéthylène glycols, les sucroglycérides d'huiles de palme et l'éthyldiéthylèneglycol rectifié.

8. Forme galénique unitaire selon la revendication 7, caractérisée en ce que l'agent hydrodispersible non ionique non tensio-actif est un mélange de 7-glycéryl-cocoate de polyoxyéthylène glycol et de polyoxyéthylène glycol.

9. Forme galénique unitaire selon la revendication 1, caractérisée en ce que l'agent de bioadhésion est un polymère biocompatible.

10. Forme galénique unitaire selon la revendication 9, caractérisée en ce que le polymère est choisi parmi les acides carboxyvinyliques, la carboxyméthylcellulose, la carboxyméthylcellulose sodique, la méthylcellulose, l'hydroxypropylcellulose, l'hydroxypropylméthylcellulose, la gélose, le silicate d'aluminium, les carraghénates, la gomme de caroube.

11. Forme galénique unitaire selon la revendication 10, caractérisée en ce que l'agent de bioadhésion est plus particulièrement l'hydroxypropylcellulose.

12. Forme galénique unitaire selon la revendication 1, caractérisée en ce que l'agent gélifiant de l'agent lipophile est choisi parmi le groupe comprenant la silice, le monostéarate d'aluminium, le tristéarate d'aluminium, l'alcool cétylique.

13. Forme galénique unitaire selon la revendication 12, caractérisée en ce que l'agent gélifiant de l'agent lipophile est plus particulièrement la silice.

14. Forme galénique unitaire selon la revendication 1, caractérisée en ce que sa phase interne compatible avec le latex de caoutchouc d'un préservatif répond à la formule générale :
| | |
|---|---|
| Chlorure de benzalkonium, solution à 50 % | 0,265 à 2,65 g |
| Silice | 5 à 7 g |
| Hydroxypropylcellulose | 6 à 8 g |
| 7-glycéryl-cocoate de polyoxyéthylène glycol | 9 à 11 g |
| Polyoxyéthylène glycol 400 | 4 à 6 g |
| Huile de silicone | q.s. 100 g |

15. Procédé d'obtention de la forme galénique unitaire selon la revendication 14, caractérisé en ce qu'il consiste :
a) à ajouter sous agitation une solution de chlorure de benzalkonium à de l'huile de silicone,
b) à incorporer au mélange a) du polyoxyéthylène glycol 400 et du 7-glycéryl-cocoate de polyoxyéthylène glycol sous forme liquide,
c) à mélanger le produit b) selon une durée suffisante pour obtenir une bonne homogénéisation,
d) à ajouter au produit c) de l'hydroxypropylcellulose,
e) à mélanger le produit d),
f) à incorporer de la silice et
g) à agiter jusqu'à obtention du produit final, qui est introduit dans l'enveloppe comprenant de la gélatine.

## Claims

1. A unit galenical formulation for vaginal use as a local contraceptive and/or to combat sexually-transmitted diseases and/or HIV, comprising firstly an outer envelope including gelatin and secondly a non-aqueous liquid or semi-liquid internal phase containing an active principle in solution, the formulation being characterized in that the active principle is constituted by a spermicide and in that the internal phase includes, in addition to the active principle: a major proportion of a lipophilic agent compatible with the rubber latex of a condom; a minor proportion of at least one hydrodispersible agent; at least one bioadhesion agent; and at least one agent for gelling the lipophilic agent.

2. A unit galenical formulation according to claim 1, characterized in that the spermicide is selected from benzalkonium chloride, benzethonium chloride, cetyl pyridinium chloride, methylbenzethonium chloride, tetradecyltrimethyl ammonium bromide, benzalkonium bromide, nonylphenyl ethers, lauryl ethers, and octoxynols.

3. A unit galenical formulation according to claim 2, characterized in that the spermicide is more particularly of cationic form.

4. A unit galenical formulation according to claim 3, characterized in that the spermicide is benzalkonium chloride.

5. A unit galenical formulation according to claim 1, characterized in that the lipophilic agent compatible with the rubber latex of a condom is a silicone oil.

6. A unit galenical formulation according to claim 1, characterized in that the hydrodispersible agent is a non-ionic non-surface-active compound.

7. A unit galenical formulation according to claim 6, characterized in that the non-ionic, non-surface-active hydrodispersible agent is selected from the group comprising: esters of fatty acids and of polyols; lauric alcohol and polyethylene glycol ether; polyoxyethylene castor oil; polyoxyethylene glycerides; polyoxyethylene glycols; sucroglycerides of palm oils; and rectified ethyldiethyleneglycol.

8. A unit galenical formulation according to claim 7, characterized in that the non-ionic non-surface-active hydrodispersible agent is a mixture of polyoxyethylene glycol and of 7-glyceryl-cocoate of polyoxyethylene glycol.

9. A unit galenical formulation according to claim 1, characterized in that the bioadhesion agent is a biocompatible polymer.

10. A unit galenical formulation according to claim 9, characterized in that the polymer is selected from: carboxyvinyl acids; carboxymethylcellulose; sodium carboxymethylcellulose; methylcellulose; hydroxypropylcellulose; hydroxypropylmethylcellulose; agar-agar; aluminum silicate; carrageenates; and carob gum.

11. A unit galenical formulation according to claim 10, characterized in that the bioadhesion agent is more particularly hydroxypropylcellulose.

12. A unit galenical formulation according to claim 1, characterized in that the agent for gelling the lipophilic agent is selected from the group comprising: silica; aluminum monostearate; aluminum tristearate; and cetyl alcohol.

13. A unit galenical formulation according to claim 12, characterized in that the agent for gelling the lipophilic agent is more particularly silica.

14. A unit galenical formulation according to claim 1, characterized in that its internal phase compatible with the rubber latex of a condom satisfies the general formula:
| | |
|---|---|
| Benzalkonium chloride, 50% solution | 0.265 g to 2.65 g |
| Silica | 5 g to 7 g |
| Hydroxypropylcellulose | 6 g to 8 g |
| 7-glyceryl-cocoate of polyoxyethylene glycol | 9 g to 11 g |
| Polyoxyethylene glycol 400 | 4 g to 6 g |
| Silicone oil | q.s. 100 g |

15. A method of obtaining a unit galenical formulation according to claim 14, characterized in that it consists:
a) in adding a solution of benzalkonium chloride to silicone oil while stirring;
b) in incorporating polyoxyethylene glycol 400 and 7-glyceryl-cocoate of polyoxyethylene glycol in liquid form to the mixture a);
c) in mixing the product b) for sufficient time to obtain good homogenization;
d) in adding hydroxypropylcellulose to the product c);
e) in mixing the product d);
f) in incorporating the silica; and
g) in stirring until the final product is obtained which is inserted into the envelope comprising gelatin.

## Patentansprüche

1. Galenische Zubereitungseinheit zum vaginalen Gebrauch als lokales Kontrazeptivum und/oder zur Bekämpfung sexuell-übertragbarer Krankheiten und/oder HIV, die zum einen eine äußere, Gelatine enthaltende Hülle, und zum anderen eine nicht-wäßrige, flüssige oder semi-liquide innere Phase, die ein aktives Wirkprinzip in Lösung enthält, umfaßt, gekennzeichnet dadurch, daß das aktive Wirkprinzip durch ein Spermizid gebildet wird und daß die innere Phase zusätzlich zu dem aktiven Wirkprinzip enthält: einen größeren Anteil eines mit dem Latex-Kautschuk eines Kondoms verträglichen, lipophilen Agens; einen kleineren Anteil mindestens eines hydro-dispergierbaren Agens; mindestens ein Bioadhäsions-Agens und mindestens ein Geliermittel für das lipophile Agens.

2. Galenische Zubereitungseinheit nach Anspruch 1, gekennzeichnet dadurch, daß das Spermizid ausgewählt ist aus: Benzalkoniumchlorid, Benzethoniumchlorid, Cetylpyridiniumchlorid, Methylbenzethoniumchlorid, Tetradecyltrimethylammoniumbromid, Benzalkoniumbromid, Nonylphenylethern, Laurylethern und Octoxynolen.

3. Galenische Zubereitungseinheit nach Anspruch 2, gekennzeichnet dadurch, daß das Spermizid insbesondere kationischer Natur ist.

4. Galenische Zubereitungseinheit nach Anspruch 3, gekennzeichnet dadurch, daß das Spermizid Benzalkoniumchlorid ist.

5. Galenische Zubereitungseinheit nach Anspruch 1, gekennzeichnet dadurch, daß das mit dem Latex-Kautschuk eines Kondoms verträgliche, lipophile Agens ein Silikonöl ist.

6. Galenische Zubereitungseinheit nach Anspruch 1, gekennzeichnet dadurch, daß das hydro-dispergierbare Agens eine nicht-ionische, nicht-oberflächenaktive Verbindung ist.

7. Galenische Zubereitungseinheit nach Anspruch 6, gekennzeichent dadurch, daß das hydro-dispergierbare, nicht-ionische, nicht-oberflächenaktive Agens ausgewählt ist aus: Estern von Fettsäuren und von Polyolen, Äthern des Laurylalkohols und von Polyethylenglykol, polyoxyethyliertem Riziniusöl, Polyoxyethylenglyceriden, Polyoxyethylenglykolen, Sucroglyceriden von Palmölen und rektifiziertem Ethyldiethylenglykol.

8. Galenische Zubereitungseinheit nach Anspruch 7, gekennzeichnet dadurch, daß das nicht-ionische, nicht-oberflächenaktive, hydro-dispergierbare Agens ein Gemisch ist aus Polyoxyethylenglykol und dem 7-Glyceryl-cocoat von Polyoxyethylenglykol.

9. Galenische Zubereitungseinheit nach Anspruch 1 gekennzeichnet dadurch, daß das Bioadhäsions-Agens ein biokompatibles Polymer ist.

10. Galenische Zubereitungseinheit nach Anspruch 9, gekennzeichnet dadurch, daß das Polymer ausgewählt ist aus: Carboxyvinylsäuren, Carboxymethylcellulose, Natriumcarboxymethylcellulose, Methylcellulose, der Hydroxypropylcellulose, Hydroxypropylmethylcellulose, Agar-Agar, Aluminiumsilikat, Carragheenaten und Johannisbrotgummi.

11. Galenische Zubereitungseinheit nach Anspruch 10, gekennzeichnet dadurch, daß das Bioadhäsions-Agens insbesondere Hydroxypropylcellulose ist.

12. Galenische Zubereitungseinheit nach Anspruch 1, gekennzeichnet dadurch, daß das Geliermittel für das lipophile Agens ausgewählt ist aus: Kieselgel, Aluminium-monostearat, Aluminium-tristearat und Cetylalkohol.

13. Galenische Zubereitungseinheit nach Anspruch 12, gekennzeichnet dadurch, daß das Geliermittel für das lipophile Agens insbesondere Kieselgel ist.

14. Galenische Zubereitungseinheit nach Anspruch 1, gekennzeichnet dadurch, daß ihre mit dem Latex-Kautschuk eines Kondomes verträgliche, innere Phase folgende Zusammensetzung aufweist:
| | |
|---|---|
| Benzalkoniumchlorid, 50%-ige Lösung | 0,265 bis 2,65 g |
| Kieselgel | 5 bis 7 g |
| Hydroxypropylcellulose | 6 bis 8 g |
| 7-Glyceryl-cocoat von Polyoxyethylenglykol | 9 bis 11 g |
| Polyoxyethylenglykol 400 | 4 bis 6 g |
| Silikonöl | q.s. 100 g |

15. Verfahren zur Herstellung einer galenischen Zubereitungseinheit nach Anspruch 14, gekennzeichnet durch folgende Schritte:
a) Hinzufügen einer Lösung von Benzalkoniumchlorid zu Silikonöl unter Rühren;
b) Versetzen des Gemischs a) mit Polyoxyethylenglykol 400 und 7-Glyceryl-cocoat von Polyoxyethylenglykol in flüssiger Form;
c) Mischen des Produkts b) während einer Zeit, die ausreichend zur Erzielung einer guten Homogenisierung ist;
d) Hinzufügen von Hydroxypropylcellulose zu Produkt c);
e) Mischen von Produkt d);
f) Versetzen mit Kieselgel, und
g) Rühren bis zum Erhalt des Endproduktes, welches in die Gelatine enthaltende Hülle eingebracht wird.
